# EUROPÄISCHE PATENTSCHRIFT

(11) **EP 0 957 784 B1**
(45) Veröffentlichungstag und Bekanntmachung des Hinweises auf die Patenterteilung: **21.05.2003**
(21) Anmeldenummer: 98908039.5
(22) Anmeldetag: 02.02.1998
(51) Int. Cl.: A61B 17/17

(54) **ZIELGERÄT FÜR EIN IMPLANTAT ZUR VERSORGUNG TROCHANTERER UND SUBTROCHANTERER FRAKTUREN**
IMPLANT DELIVERY DEVICE IN THE TREATMENT OF TROCHANTER AND SUBTROCHANTER FRACTURES
INSTRUMENT DE GUIDAGE POUR UN IMPLANT DESTINE A TRAITER DES FRACTURES TROCHANTERIENNES ET SOUSTROCHANTERIENNES

(30) Priorität: 03.02.1997 DE 19703987
(43) Veröffentlichungstag der Anmeldung: 24.11.1999
(73) Patentinhaber: Intraplant AG, CH-6330 Cham (CH)
(72) Erfinder: HÄSSIG, Christoph, CH-5036 Oberentfelden (CH); HABEGGER, Christian, CH-5040 Schöftland (CH); SCHMOTZER, Hans, CH-5742 Kölliken (CH)
(74) Vertreter: Meissner, Bolte & Partner
(86) Internationale Anmeldenummer: EP9800546
(87) Internationale Veröffentlichungsnummer: WO98033442

(56) Entgegenhaltungen:
- EP-A- 0 736 286
- EP-B- 0 496 950
- FR-A- 2 647 006

## Beschreibung

Die Erfindung betrifft ein Zielgerät für ein Implantat zur Versorgung trochanterer und subtrochanterer Frakturen gemäß dem Oberbegriff des Anspruches 1.

Aus der EP 0 496 950 B1 ist ein Zielgerät für ein Implantat zur Versorgung trochanterer und subtrochanterer Frakturen bekannt, welches einen Kopf umfaßt, der einen Zapfen zum Halten eines in den Markraum einführbaren Femurnagels aufweist. Des weiteren umfaßt dieses Zielgerät einen seitlich am Kopf befestigten Zielarm, der sich etwa parallel zum Femurnagel erstreckt und mindestens zwei auf proximale Schrägbohrungen des Femurnagels für eine Schenkelhalsschraube ausgerichtete Schrägzielbohrungen und mindestens eine auf eine distale Querbohrung des Femurnagels für eine distale Verriegelungsschraube ausgerichtete Querzielbohrung zur Aufnahme einer Bohrhülse aufweist. Der Kopf ist bei der bekannten Ausführungsform unlösbar am Zielarm befestigt. Des weiteren weist der Kopf Arretiermittel zur lösbaren Befestigung sowie eine Schlagfläche zum Eintreiben des Femurnagels auf. Der Zielarm weist mehrere Schrägzielbohrungen auf, die unterschiedliche Winkel mit der Zielarmachse einschließen und zumindest zum Teil von der Querzielbohrung gekreuzt werden. Durch diese Konstruktion lassen sich alle Zielbohrungen in einem relativ kurzen Abschnitt des Zielgerätes unterbringen, so daß das Zielgerät entsprechend kompakt ausgeführt werden kann.

Nachteilig bei der bekannten Konstruktion ist die starre Zuordnung sämtlicher Zielbohrungen zueinander. Das bekannte Zielgerät erlaubt insbesondere keine Anpassung der Querzielbohrungen an unterschiedliche distale Querbohrungen des Femurnagels.
Dabei ist zu bedenken, daß moderne Femurnägel im distalen Bereich Langloch-Querbohrungen aufweisen, um entweder eine statische oder dynamische Verriegelung zu erhalten. Dafür eignet sich das bekannte Zielgerät aufgrund der statischen Zuordnung der Zielbohrungen zueinander nicht.

Der vorliegenden Erfindung liegt die Aufgabe zugrunde, ein Zielgerät der eingangs genannten Art zu schaffen, welches bezüglich der Querzielbohrungen mehr Freiheitsgrade besitzt.

Diese Aufgabe wird erfindungsgemäß durch die kennzeichnenden Merkmale des Anspruches 1 gelöst, wobei bevorzugte konstruktive Details in den Unteransprüchen angegeben sind.

Die erfindungsgemäße Konstruktion nach Patentanspruch 1 zeichnet sich zum einen dadurch aus, daß sie extrem kompakt ausgeführt werden kann, wobei sich zu diesem Zweck zumindest der distale Schrägzieldurchgang im Zielarm mit dem Durchgang für das plattenförmige Einsatzteil bzw. die Einsetzplatte, in der die Querzielbohrungen ausgebildet sind, kreuzt.

Besonders vorteilhaft ist daß die Querzielbohrungen sich parallel zur Längsmittenachse der Einsetzplatte, jedoch in unterschiedlichem Abstand von dieser erstrecken. Damit ist es möglich, durch Wenden der Einsetzplatte um 180 Grad die Querzielbohrungen entweder etwas weiter oben oder etwas weiter unten einzustellen, so daß bei Ausbildung von Langloch-Querbohrungen im distalen Bereich eines zugeordneten Femurnagels entweder eine statische oder dynamische Verankerung desselben möglich ist. Eine wendbare Zielbüchse mit Bohrung ist in EP-A-736 286 beschrieben.

Die Schrägzieldurchgänge, die auf korrespondierende proximale Schrägdurchgänge des Femurnagels ausgerichtet sind, dienen zur Aufnahme einer Einschlag- und/oder Bohrhülse. Eine Einschlaghülse wird immer dann verwendet, wenn das Schenkelhals-Stabilisationselement eine Schenkelhalsklinge ist, die in den Schenkelhalsknochen eingeschlagen und nicht eingeschraubt wird, so wie dies beim Stand der Technik vorgeschlagen wird.

Vorzugsweise weisen die Schrägzieldurchgänge wenigstens über einen Teil ihrer Länge jeweils einen asymmetrischen Querschnitt, insbesondere an wenigstens einer Seite abgeflachten Kreisquerschnitt auf, so daß eine Einschlaghülse drehfest plaziert werden kann. Die Abflachung der Schrägzieldurchgänge erhält man bei einer speziellen Ausführungsform dadurch, daß eine entsprechende Bohrung im Zielarm an wenigstens einer Seite über eine vorbestimmte Länge offen ausgebildet und diese Öffnung durch eine ebenflächige Platte verschließbar ist. Die Einschlaghülse hat dann einen entsprechend bzw. komplementär gestalteten Umriß in Richtung quer zur Längserstreckung. Durch die Einschlaghülse hindurch läßt sich die erwähnte Schenkelhalsklinge in den Schenkelhalsknochen eintreiben.

Die Maßnahme nach Anspruch 2, wonach die dem Femurnagel zugewandte Stirnseite der Einsetzplatte ausgehend von deren Längsmittenachse jeweils in Richtung zu den beiden schmalen Längsseiten hin dachartig geneigt ist, dienen zur Vermeidung von Kollisionen zwischen der Einsetzplatte und dem Femur. Dabei ist zu bedenken, daß die Einsetzplatte länger als der korrespondierende Durchgang im Zielarm ausgebildet ist, so daß femurseitig die Einsetzplatte über dem Zielarm vorsteht, und zwar in einem von 90 Grad abweichenden Winkel relativ zur Zielarmachse. Der Winkel der dachartigen Abschrägung relativ zur Längsmittenachse der Einsetzplatte entspricht vorzugsweise dem Winkel zwischen der Zielarmachse und der Längsmittenachse des Durchgangs für die Einsetzplatte im Zielarm.

Die Maßnahmen nach den Ansprüchen 3 und 4 dienen bei exzentrischer Anordnung der Querzielbohrungen relativ zur Längsmittenachse der Einsetzplatte zum sofortigen Erkennen der Exzentrizität dieser Querzielbohrungen.

Die Merkmale des Anspruches 5 erlauben eine funktionssichere Fixierung der Einsetzplatte innerhalb des zugeordneten Durchgangs im Zielarm.

Es versteht sich von selbst, daß die schrägen Zieldurchgänge 12, 13 als kreisrunde Bohrungen oder ovalbohrungen ausgeführt sein können, so daß auf die Verschlußplatte 16 verzichtet werden kann. In diesem Fall wäre der Zielarm 10 einstückig ausgebildet.

Weitere Merkmale und Vorteile des erfindungsgemäßen Zielgerätes werden anhand eines Ausführungsbeispiels, welches in der anliegenden Zeichnung schematisch dargestellt ist, näher erläutert.
Es zeigen:
- Figur 1: einen Teil, nämlich den distalen Teil eines Zielarms gemäß Erfindung in schematischer Seitenansicht;
- Figuren 2a und 2b: eine erste Ausführungsform einer erfindungsgemäß ausgebildeten Einsetzplatte, hier Exzenter-Platte, in Seitenansicht, Stirnansicht und perspektivischer Ansicht;
- Figur 3: den distalen Bereich des in Figur 1 dargestellten Zielarms in Draufsicht von lateral;
- Figur 4: eine zweite Ausführungsform eines erfindungsgemäß ausgebildeten Zielarms in schematischer Seitenansicht;
- Figur 5: den Zielarm gemäß Figur 4 in Draufsicht von lateral;
- Figur 6: den Zielarm gemäß den Figuren 4 und 5 in perspektivischer Ansicht;
- Figuren 8 - 10: eine zweite Ausführungsform einer erfindungsgemäß ausgebildeten Einsetzplatte, nämlich Exzenter-Platte, in flachseitiger Ansicht, perspektivischer Ansicht, im Schnitt Längslinie A-A und stirnseitiger Ansicht.

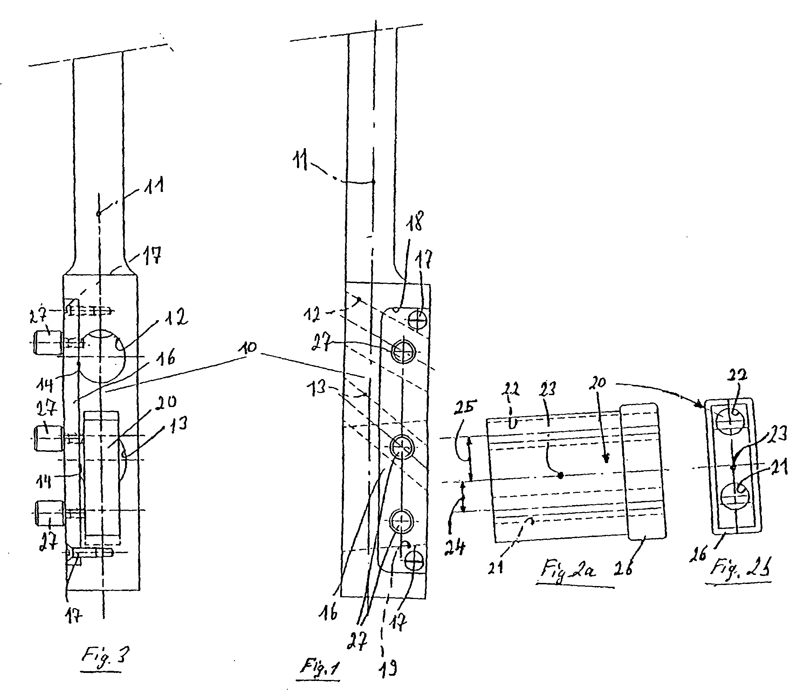

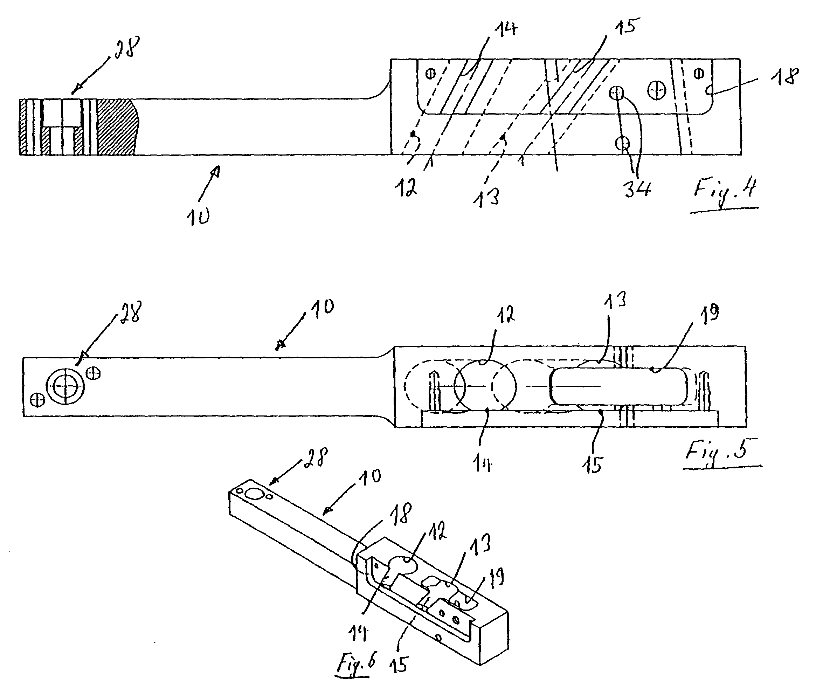

In den Figuren 1 und 3 ist der distale Abschnitt eines Zielarms 10 in Seiten- und Frontansicht dargestellt, der im proximalen Bereich mit einem Kopf lösbar verbunden ist. An diesem Kopf ist ein Femurnagel anschließbar. Bei dem vorgenannten Kopf und der Verbindung mit einem Femurnagel handelt es sich um an sich bekannte Maßnahmen, so daß eine Erläuterung hier entbehrlich ist. Es sei jedoch darauf hingewiesen, daß die lösbare Verbindung zwischen Kopf und Zielarm vorteilhaft ist dahingehend, daß beide Teile aus unterschiedlichen Materialien hergestellt werden können, z. B. der Kopf aus Kunststoff. Röntgentechnisch hat diese Konstruktion und zweiteilige Ausführung entsprechende Vorteile. Das Röntengenbild wird durch den aus Kunststoff bestehenden Zielarm nicht gestört. Die Befestigung des Zielarms 10 an dem erwähnten Kopf erfolgt so, daß die Längsachse 11 des Zielarms sich etwa parallel zu einem am Kopf befestigbaren Femurnagel erstreckt.

Im Zielarm 10 sind erste Zieldurchgänge 12, 13 ausgebildet, die verschiedene Winkel mit der Zielarmachse 11 von vorzugsweise 125 Grad, 130 Grad oder 135 Grad einschließen. Es ist auch ein Winkel von 140 Grad denkbar. Im vorliegenden Fall sind zwei Schrägzieldurchgänge 12, 13 vorgesehen. Es ist auch denkbar, mehr als zwei Schrägzieldurchgänge vorzusehen. Diese Schrägzieldurchgänge dienen der Aufnahme einer hier nicht näher dargestellten Einschlaghülse zum Einschlagen eines im Querschnitt konturierten Schenkelhals-Stabilisationselementes, insbesondere Schenkelhalsklinge mit Doppel-T-Querschnittsprofil, Sternprofil, T-Profil oder dergleichen. Der jeweilige Schrägzieldurchgang wird entsprechend der Ausrichtung eines proximalen Schrägdurchgangs im nach anatomischen Gegebenheiten ausgewählten Femurnagel gewählt. Beide Schrägzieldurchgänge 12 und 13 weisen wenigstens über einen Teil ihrer Länge (siehe dazu insbesondere Figur 6) jeweils einen asymmetrischen, nämlich an einer Seite abgeflachten Kreisquerschnitt auf zur drehfesten Plazierung der erwähnten Einschlaghülse, dessen Außenkontur dem erwähnten Querschnitt der Zieldurchgänge 12 und 13 entspricht. Die Abflachung der Schrägzieldurchgänge 12, 13 wird dadurch erhalten, daß eine entsprechende Bohrung im Zielarm 10 so gesetzt wird, daß diese an wenigstens einer, in Figur 3 linken Seite über eine vorbestimmte Länge (siehe Figur 6) offen ausgebildet und diese Öffnung 14, 15 durch eine ebenflächige Platte 16 verschließbar ist. Die Platte 16 wird mittels Schrauben 17 am Zielarm 10, und zwar innerhalb einer entsprechenden Ausnehmung 18 fixiert. Die Fixierung innerhalb der Ausnehmung 18 erfolgt so, daß die Platte 16 außenseitig bündig mit dem Zielarm 10 abschließt.

Der Zielarm 10 hat ferner einen zweiten Durchgang 19, der entsprechend Figur 1 ebenfalls schräg zur Zielarm-Längsachse 11 geneigt ist, und zwar entgegengesetzt zu der Neigung der ersten Schrägzieldurchgänge 12 und 13. Der Durchgang 19 ist so angeordnet, daß er den distalen Schrägzieldurchgang 13 kreuzt bzw. schneidend durchsetzt. Der Schrägdurchgang 19 dient zur Aufnahme eines plattenförmigen Einsetzteils bzw. einer Einsetzplatte 20, die sich parallel zur Längsachse der Einsetzplatte 20 erstreckende Querzielbohrungen 21, 22 umfaßt, die im montierten Zustand der Einsetzplatte 20 auf distale Querbohrungen des hier nicht dargestellten Femurnagels gerichtet sind. In der Regel hat jeder Femurnagel zwei distale Querbohrungen, wobei moderne Femurnägel Langloch-Querbohrungen aufweisen, so daß der Femurnagel entweder statisch oder dynamisch im Femur fixierbar ist. Bei statischer Fixierung liegen die Knochenschrauben am proximalen Ende der Langloch-Querbohrungen an, während sie bei dynamischer Fixierung am distalen Ende der erwähnten Langloch-Querbohrungen anliegen. Um eine entsprechende Plazierung der Knochenschrauben zu ermöglichen, sind die Querzielbohrungen 21, 22 relativ zur Längsmittenachse 23 der Einsetzplatte 20 unterschiedlich weit von der Längsmittenachse 23 beabstandet. Durch Wenden der Einsetzplatte 20 um 180 Grad lassen sich somit die Querzielbohrungen 21, 22 von unten nach oben verlagern und umgekehrt. Die unterschiedlichen Abstände der Längsachsen der Querzielbohrungen 21, 22 von der Längsmittenachse 23 der Einsetzplatte 20 sind in Figur 2a mit den Abstandspfeilen 24, 25 dargestellt. Die Einsetzplatte dieser Art kann somit auch als Exzenter-Platte bezeichnet werden, da die Querzielbohrungen 21, 22 relativ zur Längsmittenachse 23 exzentrisch angeordnet sind.

Um ein zu weites Einschieben der Einsetzplatte 20 in den Schrägdurchgang 19 des Zielarms 10 von lateral her zu vermeiden, weist die Einsetzplatte 20 an ihrem lateralen Stirnende einen nach außen vorspringenden Bund 26 auf. Dieser Bund 26 dient als Anschlag an der lateralen Seite des Zielarms 10 beim Einschieben der Einsetzplatte 20 in den Durchgang 19 des Zielarmes 10 von lateral her.

Nach Montage der Einsetzplatte 20 ist nur noch der proximale Schrägdurchgang 12 zugänglich. Für den Fall, daß der distale Schrägzieldurchgang 13 verwendet werden soll, muß die Einsetzplatte 20 entfernt werden.

Der Vorteil der Einsetzplatte 20 besteht darin, daß in dieser ein, zwei, drei oder mehr Querzielbohrungen parallel oder im Winkel zueinander ausgebildet sein können. Auch ist es denkbar, Einsetzplatten mit Querzielbohrungen unterschiedlichen Durchmessers zur Verfügung zu stellen. Auch können Einsetzplatten mit Querzielbohrungen zur Verfügung gestellt werden, die einen unterschiedlichen Abstand von der Längsmittenachse 23 der Einsetzplatte 20 aufweisen. Die Variabilität der beschriebenen Konstruktion ist also im Vergleich zu der starren Ausführung gemäß dem eingangs erwähnten Stand der Technik erheblich verbessert.

Die Einsetzplatte 20 wird bei der Ausführungsform nach den Figuren 1 bis 3 durch Klemmschrauben 27 fixiert, die in entsprechende Gewindebohrungen der Platte 16 einschraubbar sind. Den Schrägzieldurchgängen 12 und 13 sind ebenfalls Klemmschrauben zugeordnet entsprechend den Klemmschrauben 27, wie insbesondere Figur 1 entnommen werden kann. Die in Figur 1 mittlere Klemmschraube 27 hat insofern eine Doppelfunktion. Sie dient zum einen zum Festklemmen der Einsetzplatte 20 und zum anderen zum Festklemmen einer in den distalen Schrägzieldurchgang 13 eingeführten Einschlaghülse.

Die Ausführungsform nach den Figuren 4 bis 6 entspricht im wesentlichen derjenigen nach den Figuren 1 bis 3, wobei Figur 6 sehr schön die Längsöffnungen der Schrägzieldurchgänge 12 und 13 erkennen läßt, die dann durch die erwähnte Platte 16 verschließbar sind. Auch lassen die Figuren 4 bis 6 das proximale Ende des Zielarms 10 erkennen mit einer Einrichtung 28 zum Anschluß des oben erwähnten Kopfes. Figur 6 läßt des weiteren gut die gegenseitige Verschneidung zwischen dem Durchgang 19 einerseits und dem distalen Schrägzieldurchgang 13 erkennen.

Bei der Ausführungsform nach den Figuren 4 bis 6 sind die Längskanten des Durchganges 19 abgerundet. Natürlich muß dann auch die Einsetzplatte 20 entsprechend abgerundete oder fasetierte Längskanten aufweisen.

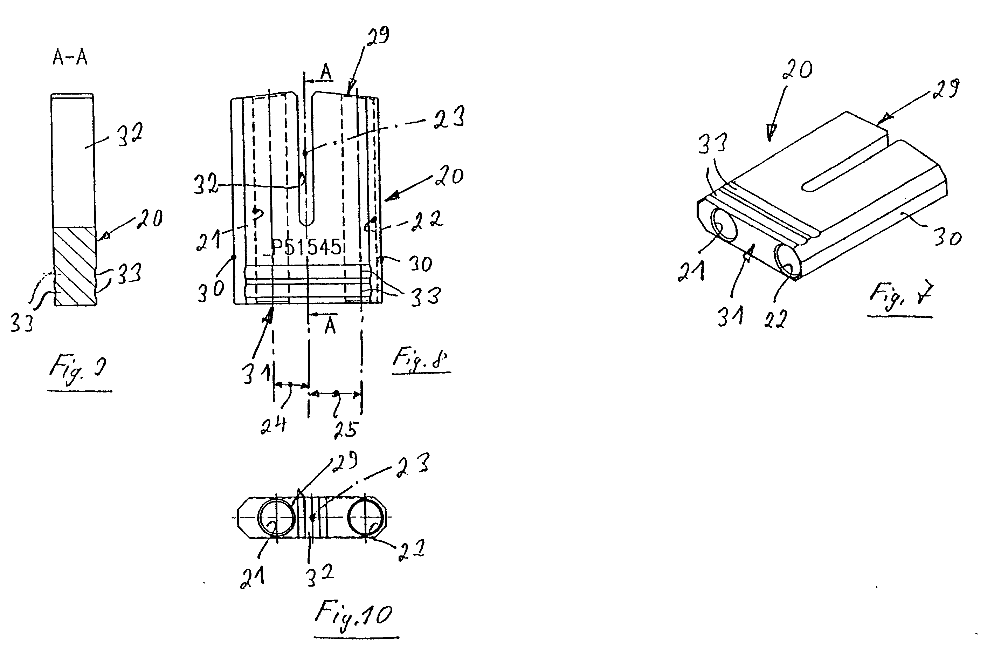

Die der Ausführungsform nach den Figuren 4 bis 6 zugeordnete Einsetzplatte ist in den Figuren 7 bis 10 dargestellt. Sie hat wiederum die Bezugsziffer 20. Die Querzielbohrungen sind mit den Bezugsziffern 21, 22 gekennzeichnet. Die Längsmittenachse der Einsetzplatte 20 ist auch in Figur 8 und 10 mit der Bezugsziffer 23 angegeben. Die Querzielbohrungen 21, 22 sind relativ zur Längsmittenachse 23 der Einsetzplatte 20 unterschiedlich weit von der Längsmittenachse 23 entfernt. Die entsprechenden Entfernungspfeile sind auch hier mit den Bezugsziffern 24 und 25 versehen.

Die dem Femurnagel zugewandte Stirnseite 29 der Einsetzplatte 20 ist bei der in den Figuren 7 bis 10 dargestellen Ausführungsform ausgehend von der Längsmittenachse 23 jeweils in Richtung zu den beiden schmalen Längsseiten 30 hin dachartig geneigt, und zwar in Richtung nach lateral. Damit läßt sich trotz schrägen Einsatzes der Einsetzplatte 29 eine Kollision des inneren Endes mit dem Patienten vermeiden. Der Winkel der dachartigen Abschrägung relativ zur Längsmittenachse 23 entspricht in etwa dem Winkel der Längsmittenachse des Durchgangs 19 relativ zur Längsachse 11 des Zielarms 10.

Die Einsetzplatte 20 hat bei der in den Figuren 7 bis 10 dargestellten Ausführungsform auch noch einen sich ausgehend von der dem Femurnagel zugewandten Stirnseite 29 in Richtung zu der dem Femurnagel abgewandten Stirnseite 31 hin erstreckenden Einschnitt bzw. Längsschlitz 32, der sich in Richtung der Längsmittenachse 23 erstreckt und zwar über etwa 2/3 der Gesamtlänge der Einsetzplatte 20. Dieser Längsschlitz korrespondiert beim Einstecken der Einsetzplatte 20 in den Zielarm-Durchgang 19 mit sich durch diesen hindurcherstreckenden Führungsstiften 34. Diese liegen in Einsteckrichtung bzw. in Richtung parallel zur Längsachse des Durchgangs 19 im Abstand hintereinander. Sie halten die Einsetzplatte spielfrei im Durchgang und erleichtern das Ein- und Ausführen der Einsetzplatte ohne Gefahr einer Verklemmung beim Ein- und Ausführen derselben.

Am lateralen Ende ist die Einsetzplatte 20 an wenigstens einer, hier beiden Flachseiten mit Griffnuten 33 oder dgl. Griffmulden bzw. Griffhilfen versehen, die die Handhabung erleichtern. Die Fixierung der Einsetzplatte 20 im Durchgang 19 erfolgt durch die bereits erwähnten Klemmschrauben 27.

### Bezugszeichenliste:

- 10: Zielarm
- 11: Zielarm-Längsachse
- 12: Schrägzieldurchgang
- 13: Schrägzieldurchgang
- 14: Öffnung
- 15: Öffnung
- 16: Platte
- 17: Schrauben
- 18: Ausnehmung
- 19: Schrägdurchgang
- 20: Einsetzplatte
- 21: Querzielbohrung
- 22: Querzielbohrung
- 23: Längsmittenachse
- 24: Abstandspfeil
- 25: Abstandspfeil
- 26: umlaufender Bund
- 27: Klemmschraube
- 28: Kopf-Anschlußeinrichtung
- 29: Stirnseite
- 30: Längsseite (schmal)
- 31: Stirnseite
- 32: Längsschlitz
- 33: Griffnuten
- 34: Führungsstifte

## Patentansprüche

1. Zielgerät für ein Implantat zur Versorgung trochanterer und subtrochanterer Frakturen mit einem Kopf zum Anschluß eines Femurnagels und mit einem seitlich am Kopf befestigten Zielarm (10), der sich etwa parallel zum Femurnagel erstreckt und mindestens zwei auf einen proximalen Schrägdurchgang des Femurnagels für eine Schenkelhalsklinge oder Schenkelhals-Stabilisationselement ausgerichtete Schrägzieldurchgänge (12, 13) und mindestens zwei auf eine distale Querbohrung des Femurnagels ausgerichtete Querzielbohrungen (21,22) zur Aufnahme einer Einschlagund/oder Bohrhülse aufweist, wobei die Schrägzieldurchgänge (12, 13) unterschiedliche Winkel mit der Zielarmachse (11) einschließen und die Längsachsen der Schrägzieldurchgänge (12, 13) die Längsachsen der Querzielbohrungen (21, 22) kreuzen, **dadurch gekennzeichnet, daß** die Querzielbohrungen (21, 22) Teil eines plattenförmigen Einsetzteils (20) sind, das innerhalb eines korrespondierenden Durchgangs (19) im Zielarm (10) plazier- und fixierbar ist, und sich die Querzielbohrungen parallel zur Längsmittenachse (23) des plattenförmigen Einsatzteils (20), jedoch in unterschiedlichem Abstand von dieser erstrecken, so daß durch Wenden der Einsetzplatte um 180 Grad die Querzielbohrungen (21, 22) verschiebbar sind.

2. Zielgerät nach Anspruch 1,
**dadurch gekennzeichnet, daß**
die dem Femurnagel zugewandte Stirnseite (29) der Einsetzplatte (20) ausgehend von deren Längsmittenachse (23) jeweils in Richtung zu den beiden schmalen Längsseiten (30) hin dachartig geneigt ist.

3. Zielgerät nach Anspruch 1 oder 2,
**dadurch gekennzeichnet, daß**
die Einsetzplatte (20) einen ausgenend von der dem Femurnagel zugewandten Stirnseite (29) sich in Richtung zu der dem Femurnagel abgewandten Stirnseite (31) hin erstreckenden Einschnitt oder Längsschlitz (32) aufweist, der mit einem im Durchgang (19) für die Einsetzplatte (20) angeordneten Führungselement (Führungsstifte 34) für die Einsetzplatte (20) korrespondiert.

4. Zielgerät nach Anspruch 3,
**dadurch gekennzeichnet, daß**
der Längsschlitz (32) sich in Richtung der Längsmittenachse (23) der Einsetzplatte (20) erstreckt, und zwar über etwa 1/2 bis 2/3 der Gesamtlänge der Einsetzplatte (20).

5. Zielgerät nach einem der Ansprüche 1 bis 4,
**dadurch gekennzeichnet, daß**
die Einsetzplatte (20) durch wenigstens eine in den Zielarm (10) einschraubbare Klemmschraube (27) fixierbar ist.

6. Zielgerät nach einem der Ansprüche 1 bis 5,
**dadurch gekennzeichnet, daß**
der Zielarm (10) und der Kopf zum Anschluß des Femurnagels lösbar miteinander verbunden sind.

7. Zielgerät nach einem der Ansprüche 1 bis 6,
**dadurch gekennzeichnet, daß**
die Schrägzieldurchgänge (12, 13) jeweils wenigstens über einen Teil ihrer Länge einen asymmetrischen Querschnitt, inbesondere an wenigstens einer Seite abgeflachten Kreisquerschnitt aufweisen zur drehfesten Plazierung einer Einschlag- und/oder Bohrhülse.

8. Zielgerät nach Anspruch 7,
**dadurch gekennzeichnet, daß**
die Abflachung der Schrägzieldurchgänge (12, 13) dadurch erhalten ist, daß eine entsprechende Bohrung im Zielarm (10) an wenigstens einer Seite über eine vorbestimmte Länge offen ausgebildet und diese Öffnung (14, 15) durch eine ebenflächige Platte (16) verschließbar ist.

## Claims

1. An aiming device for an implant for the treatment of trochanteric and subtrochanteric fractures, having a head for connecting a femur nail and having an aiming arm (10), which is fixed laterally to the head, extends approximately parallel to the femur nail and has at least two inclined aiming passages (12, 13) aligned with a proximal inclined passage of the femur nail for a femur neck blade or femur neck stabilising element, and at least two transverse aiming bores (21, 22) aligned with a distal transverse bore of the femur nail for the purpose of receiving an impact and/or drill sleeve, the inclined aiming passages (12, 13) forming different angles with the aiming arm axis (11) and the longitudinal axes of the inclined aiming passages (12, 13) intersecting the longitudinal axes of the transverse aiming bores (21, 22), **characterised in that** the transverse aiming bores (21, 22) form part of a plate-shaped insert part (20), which may be positioned and fixed within a corresponding passage (19) in the aiming arm (10), and the transverse aiming bores extend parallel to the longitudinal centre axis (23) of the plate-shaped insert part (20), although at a different spacing therefrom, so that the transverse aiming bores (21, 22) may be displaced by turning the insert plate through 180 degrees.

2. An aiming device according to Claim 1, **characterised in that**, starting from the longitudinal centre axis (23) of the insert plate (20), that end face (29) of the insert plate (20) which faces the femur nail is inclined in each case in the direction of the two narrow longitudinal sides (30) in roof-like manner.

3. An aiming device according to Claim 1 or 2, **characterised in that** the insert plate (20) has an incision or longitudinal slot (32) which, starting from the end face (29) facing the femur nail, extends in the direction of the end face (31) remote from the femur nail and corresponds to a guide element (guide pins 34) for the insert plate (20), which is arranged in the passage (19) for the insert plate (20).

4. An aiming device according to Claim 3, **characterised in that** the longitudinal slot (32) extends in the direction of the longitudinal centre axis (23) of the insert plate (20), and more precisely over approximately 1/2 to 2/3 of the entire length of the insert plate (20).

5. An aiming device according to one of Claims 1 to 4, **characterised in that** the insert plate (20) may be fixed in position by at least one clamping screw (27) which may be screwed into the aiming arm (10).

6. An aiming device according to one of Claims 1 to 5, **characterised in that** the aiming arm (10) and the head for connecting the femur nail are detachably connected to one another.

7. An aiming device according to one of Claims 1 to 6, **characterised in that** the inclined aiming passages (12, 13) each have an asymmetrical cross-section at least over part of their length, in particular a flattened circular cross-section on at least one side, for positioning an impact and/or drill sleeve in torsion-resistant manner.

8. An aiming device according to Claim 7, **characterised in that** the flattening of the inclined aiming passages (12, 13) is achieved **in that** a corresponding bore is incorporated in the aiming arm (10) such that it is open over a predetermined length on at least one side, and this opening (14, 15) may be closed by a planar plate (16).

## Revendications

1. Viseur pour un implant destiné à soigner des fractures trochantériennes et sub-trochantériennes, avec une tête pour le raccordement d'un clou de fémur et avec un bras de visée (10) fixé latéralement sur la tête, bras qui s'étend approximativement parallèlement au clou de fémur et qui présente au moins deux passages de visée obliques (12, 13) orientés en direction d'un passage oblique proximal du clou de fémur pour une lame de col du fémur ou un élément de stabilisation de col du fémur et au moins deux perçages de visée transversaux (21, 22) orientés en direction d'un perçage transversale distal du clou de fémur pour recevoir une douille d'enfoncement et/ou de forage, les passages de visée obliques (12, 13) délimitant des angles différents avec l'axe (11) du bras de visée et les axes longitudinaux des passages de visée obliques (12, 13) coupant les axes longitudinaux des perçages de visée transversaux (21, 22), **caractérisé en ce que** les perçages de visée transversaux (21, 22) font partie d'une partie d'enfoncement tabulaire (20) qui peut être placée et fixée à l'intérieur d'un passage correspondant (19) dans le bras de visée, et les perçages de visée transversaux s'étendent parallèlement à l'axe longitudinal médian (23) de la partie d'enfoncement tabulaire (20), mais à une distance différente de celle-ci, de sorte que les perçages de visée transversaux (21, 22) peuvent être déplacés en faisant tourner la plaque d'enfoncement de 180 degrés.

2. Viseur selon la revendication 1, **caractérisé en ce que** la face frontale (29) de la plaque d'enfoncement (20) tournée vers le clou de fémur est inclinée à la manière d'un toit en partant de son axe longitudinal médian (20), en direction respectivement des deux petits côtés longitudinaux (30).

3. Viseur selon la revendication 1 ou 2, **caractérisé en ce que** la plaque d'enfoncement (20) présente une encoche ou fente longitudinale (32) partant de la face frontale (29) tournée vers le clou de fémur et s'étendant en direction de la face frontale (31) opposée au clou de fémur, encoche qui correspond à un élément de guidage (ergots de guidage 34) de la plaque d'enfoncement (20) placé dans le passage (19) destiné à la plaque d'enfoncement (20).

4. Viseur selon la revendication 3, **caractérisé en ce que** la fente longitudinale (32) s'étend en direction de l'axe longitudinal médian (23) de la plaque d'enfoncement (20), sur environ 1/3 à 2/3 de la longueur totale de la plaque d'enfoncement (20).

5. Viseur selon l'une des revendications 1 à 4, **caractérisé en ce que** la plaque d'enfoncement (20) peut être fixée par au moins une vis de blocage (27) se vissant dans le bras de visée (10).

6. Viseur selon l'une des revendications 1 à 5, **caractérisé en ce que** le bras de visée (10) et la tête sont reliés entre eux de manière amovible pour se raccorder au clou de fémur.

7. Viseur selon l'une des revendications 1 à 6, **caractérisé en ce que** les passages de visée obliques (12, 13) présentent respectivement, au moins sur une partie de leur longueur, une section transversale asymétrique, en particulier une section transversale circulaire aplatie sur au moins un côté, afin de placer une douille d'enfoncement et/ou de forage de manière immobile en rotation.

8. Viseur selon la revendication 7, **caractérisé en ce que** le méplat des passages de visée obliques (12, 13) est obtenu par le fait qu'un perçage correspondant dans le bras de visée (10) est réalisé ouvert au moins d'un côté sur une longueur prédéterminée et cette ouverture (14, 15) peut être fermée par une plaque (16) à surface plane.
